# EUROPÄISCHE PATENTSCHRIFT

(11) **EP 1 916 977 B1**
(45) Veröffentlichungstag und Bekanntmachung des Hinweises auf die Patenterteilung: **05.07.2017**
(21) Anmeldenummer: 06791669.2
(22) Anmeldetag: 25.08.2006
(51) Int. Cl.: A61F 9/009, A61F 9/01

(54) **KONTAKTGLAS FÜR DIE AUGENCHIRURGIE**
CONTACT GLASS FOR OPHTHALMIC SURGERY
VERRE DE CONTACT POUR LA CHIRURGIE OCULAIRE

(30) Priorität: 25.08.2005 DE 102005040337; 25.08.2005 DE 102005040338
(43) Veröffentlichungstag der Anmeldung: 07.05.2008
(73) Patentinhaber: Carl Zeiss Meditec AG, 07745 Jena (DE)
(72) Erfinder: MÜHLHOFF, Dirk, 07751 Kunitz (DE); EBERT, Elke, 07743 Jena (DE); FESTAG, Karsten, 07749 Jena (DE); WOLF, Uwe, 99441 Magdala (DE); PREUSS, Dirk, 07749 Jena (DE); BISCHOFF, Mark, 07749 Jena (DE); STOBRAWA, Gregor, 07743 Jena (DE); STEINMETZ, Dietmar, 07751 Bucha (DE); DUBNACK, Steffen, 07745 Jena (DE)
(74) Vertreter: Patentanwälte Geyer, Fehners & Partner mbB
(86) Internationale Anmeldenummer: PCT/EP2006/008360
(87) Internationale Veröffentlichungsnummer: WO 2007/022993

(56) Entgegenhaltungen:
- EP-A2- 1 199 046
- WO-A-2005/039462
- WO-A2-2006/066035
- DE-A1- 10 353 264
- US-A- 5 108 412
- US-A- 5 772 675
- US-A1- 2004 220 602
- US-A1- 2006 129 140

## Beschreibung

Die Erfindung bezieht sich auf ein System umfassend ein Kontaktglas für die Augenchirurgie, das eine zum Aufsetzen auf das Auge ausgebildete Linsenvorderfläche und eine Einrichtung zur Unterdruckbefestigung des Kontaktglases am Auge aufweist.

Ein solches Kontaktglas ist in der WO 2005/048895 A1 gezeigt, die sich ansonsten mit der Befestigung des Kontaktglases an einer Laserbehandlungsvorrichtung beschäftigt.

Kontaktgläser in der Augenchirurgie sind Beispiele für Adaptoren, die die Laserbearbeitungsvorrichtung mit einem Objekt mechanisch koppeln. Diese Kopplung ist erforderlich, da die Genauigkeit der Positionierung des Laserstrahls im Objekt in der Regel die bei der Bearbeitung erreichte Präzision vorgibt. Erst die exakte dreidimensionale Positionierung des Laserstrahls in dem Bearbeitungsvolumen, beispielsweise in der Hornhaut des Auges, ermöglicht eine hochgenaue Bearbeitung. Die Fixierung des zu bearbeitenden Objektes wird deshalb durch einen Adapter vorgenommen, der eine genau definierte Lage des Objektes, beispielsweise des Auges gegenüber der Laserbearbeitungsvorrichtung gewährleistet. Der Adapter, der in der Augenchirurgie üblicherweise als Kontaktglas bezeichnet wird, ist damit Teil des Strahlengangs. Ist die äußere Form des zu bearbeitenden Objektes nicht exakt bekannt, hat der Adapter zumeist auch die Funktion, dem Objekt, soweit möglich, eine bestimmte, bei der Applikation eines Laserstrahls vorausgesetzte Form zu geben.

Da die Vorderfläche der Hornhaut des menschlichen Auges von Patient zu Patient variiert, ist bei der lasergestützten Augenchirurgie regelmäßig ein Adapter in Form eines Kontaktglases vorgesehen. Die US 2001/0021844 A1 beschreibt ein entsprechendes Kontaktglas, das nicht nur die Fixierung des Auges, sondern auch eine Formgebung der Hornhautvorderfläche bewerkstelligt. Die US-Schrift schlägt vor, zwischen der Hornhaut und dem als Linsenkörper ausgeführten Kontaktglas Unterdruck anzulegen, wodurch die Augenhornhaut an das Kontaktglas gesaugt wird. Durch die derart zwischen Linsenkörper und Augenhornhaut erfolgende Unterdruckbefestigung nimmt die Augenhornhaut automatisch die Form der Linsenkörpervorderfläche (Vorderfläche ist auf den Patienten bezogen) an. Diese Art der Befestigung ist jedoch für den Patienten recht unangenehm, insbesondere wenn die in einer Ausführungsform der US 2001/0021844 A1 an der Unterseite der Linsenkörper-Fassung vorgesehenen, widerhakenartigen Noppen verwendet werden, die eine verbesserte Befestigung des Kontaktglases am Auge erreichen sollen.

Der Erfindung liegt die Aufgabe zu Grunde, ein Kontaktglas der eingangs genannten Art so fortzubilden, dass eine Überwachung der Anzahl seiner Einsätze ermöglicht wird.

Diese Aufgabe wird erfindungsgemäß mit einem System gemäß Anspruch 1 gelöst. In einer bevorzugten Ausführungsform ist am Kontaktglas die Linsenvorderfläche ringförmig von einem gegenüber einer gedachten Verlängerung der Linsenvorderfläche nicht vorstehenden Ringspalt oder von einer Vielzahl von Ansaugöffnungen umgeben, durch den/die hindurch Unterdruck auf das Auge wirkt.

Die Augenhornhaut wird durch mechanischen Druck an die Linsenvorderfläche angelegt bzw. dort gehalten, wobei der mechanische Druck aus einer Ansaugung der Augenhornhaut resultiert, die in einem die Linsenvorderfläche umgebenden ringförmigen Bereich vorgenommen wird. Der ringförmige Bereich weist Ansaugöffnungen auf, die den Unterdruck zuführen. Dieses Vorgehen erreicht zwei wesentliche Vorteile. Zum einen kann der Unterdruck auf die Augenhornhaut so schonend wie möglich aufgebracht werden. Es ist in einer Variante zudem ausgeschlossen, dass die Augenhornhaut ringförmig in einen Ringspalt hineingesaugt wird. Damit ist auch ein unerwünschtes Verstopfen des Saugkanals ausgeschlossen. Zum anderen kann die Patientenseite des Kontaktglases im wesentlichen glatt ausgebildet sein; das Auge wird an eine glatte Fläche angedrückt, die angeordnete Ansaugöffnungen hat. Scharfkantige Einwirkungen auf das Auge sind damit vermieden, bzw. der beim Stand der Technik erforderliche Fertigungsaufwand zur Vermeidung scharfer Kanten an einem Saugring entfällt nun. Die Ausgestaltung schafft also ein Kontaktglas für die Augenchirurgie, das einen in einer Fassung aufgenommenen Linsenkörper aufweist, welcher eine zum Aufsetzen auf das Auge ausgebildete, konkave Linsenvorderfläche aufweist, wobei am Rand der Linsenvorderfläche zwischen dem Linsenkörper und der Fassung ein Ringspalt bzw. ringförmiger Bereich gebildet ist, der zusammen mit dem angelegten Auge als Saugkanal wirkt, über den Unterdruck zum Befestigen des Kontaktglases am Auge applizierbar ist, wobei die Fassung gegenüber einer gedachten Verlängerung der Linsenvorderfläche nicht vorsteht. Der Ringspalt bzw. ringförmige Bereich formt zusammen mit dem angelegten Auge eine weitestgehend luftdichte Kammer, so dass mittels Unterdruck eine Kraft zur Befestigung des Kontaktglases an der Augenhornhaut applizierbar ist.

Bevorzugt ist die Fassung so ausgebildet, dass bei vollflächig an die Linsenvorderfläche angelegtem Auge der Saugkanal zwischen Ringspalt und Auge noch nicht geschlossen ist.

Im Gegensatz zu dem in US 2001/0021844 A1 beschriebenen Vorgehen, wird die Augenhornhaut nicht durch Vakuum direkt an die Linsenvorderfläche angesaugt, sondern durch mechanischen Druck an die Linsenvorderfläche angelegt. Dadurch, dass die Fassung so ausgebildet ist, dass bei vollständig an die Linsenvorderfläche angelegtem Auge der Saugkanal zwischen Ringspalt und Auge noch offen bleibt (die Augenhornhaut den Ringspalt also noch nicht abdeckt) kann in diesem Zustand eine genaue Ausrichtung des Kontaktglases gegenüber dem Auge erfolgen. Erst wenn das Kontaktglas weiter auf das Auge hin gedrückt wird, überdeckt die Augenhornhaut den Ringspalt, wodurch der Saugkanal zwischen Ringspalt und Auge geschlossen ist und die Unterdruckbefestigung wirkt. Das im Ringspalt aufgebrachte Vakuum dient somit nur zur Fixierung des Auges, bewirkt aber nicht direkt die Verformung der Augenhornhaut. Damit ist der gesamte Prozess des Anlegens und Fixierens besser kontrollierbar.

Das durch den Saugkanal aufgebrachte Vakuum dient nur zur Fixierung des Auges, bewirkt aber nicht direkt die Verformung der Augenhornhaut, da kein Vakuum zwischen der Linsenvorderfläche und der Augenhornhaut wirkt. Das erfindungsgemäße Kontaktglas ist also besonders für Patienten geeignet, die in dem Bereich der Hornhaut, der an die Linsenvorderfläche angelegt werden soll, Hornhautschäden, Anomalien oder gar nicht verheilte Schnitte früherer augenchirurgischer Eingriffe haben. Da in diesem Bereich der Augenhornhaut kein Unterdruck wirkt, ist jetzt eine schädigende Wirkung der Kontaktglasbefestigung ausgeschlossen.

Auf der Patientenseite ist der Übergang zwischen der Linsenvorderfläche und der Fassung (bis auf den Ringspalt) fließend; die Fassung bildet jenseits des Ringspaltes keine Sprungstelle.

Die Fassung ist geometrisch so gestaltet, dass die Augenhornhaut in einem Zustand, in dem das Auge bereits vollflächig an die Linsenvorderfläche angelegt ist, noch nicht am Fassungsrand, der den Ringspalt begrenzt, anliegt, folglich den Ringspalt noch nicht zum Saugkanal schließt. Dies kann beispielsweise dadurch erreicht werden, dass der axial vorderste Umriss der Fassung gegenüber einer Krümmungsfläche, die die Augenhornhaut hat, wenn das Auge vollständig an der Linsenvorderfläche anliegt, nicht vorsteht oder gegenüber dieser sogar zurückbleibt. Die Form der Krümmungsfläche hängt im wesentlichen von dem Zustand ab, den das Auge bei vollständiger Anlage an die Linsenvorderfläche hat.

In einer einfachen Gestaltung kann man für die Krümmungsfläche, gegenüber der der axial vorderste Umriss der Fassung nicht vorsteht oder sogar zurückbleibt, auch Bezug auf die Krümmung der Linsenvorderfläche nehmen, so dass dann der axial vorderste Umriss der Fassung gegenüber der gedachten Verlängerung der Linsenvorderfläche nicht vorsteht oder sogar zurückbleibt.

Mit dem Kontaktglas kann die natürliche Form der Hornhaut bei angelegtem Kontaktglas gewahrt werden, was für den Patienten besonders angenehm ist. Das Auge wird bei geeignetem Krümmungsradius mit minimalen Deformationen an das Kontaktglas gelegt.

Eine besonders einfache Fertigung erreicht man, wenn man den Ringspalt durch eine oder mehrere Kegelstumpfmantelflächen bzw. konische Flächen an Fassung und/oder Linsenkörper ausbildet. Mit solchen konischen Flächen ist darüber hinaus in der Regel eine weitere Ausführungsform einfach realisiert, bei der sich der Ringspalt vom Auge weg verjüngt und insbesondere am vom Auge abgewandten Ende unter einem spitzen Winkel ≤ 90° zusammenläuft.

Eine solche Gestaltung des Ringspaltes ist weiter vorteilhaft, weil dadurch effektiv vermieden wird, dass Teile der Augenhornhaut bis in den oberen Bereich des Saugkanals gesaugt werden und diesen dadurch teilweise verstopfen. Die Einleitung des Unterdrucks in den Ringspalt ist dann unkritisch, da immer der gesamte Ringspalt als Saugkanal wirkt und insbesondere der Unterdruckanschluss nicht durch die Augenhornhaut verschlossen werden kann.

Das Kontaktglas ermöglicht, insbesondere wenn die Krümmungsfläche der Linsenvorderfläche etwas flacher gekrümmt ist, als die Augenhornhaut, ein besonders einfaches Aufsetzen des Kontaktglases auf die Augenhornhaut. Bei Annäherung an das Kontaktglas berührt zunächst nur der zentrale Bereich der Augenhornhaut, also der Augenhornhautscheitel, die Linsenvorderfläche. Bei zunehmendem Aufsetzen liegt nach und nach die Augenhornhaut an der vollständigen Linsenvorderfläche an, wobei auch in diesem Zustand der Saugkanal noch nicht geschlossen ist und der Patient das Auge trotz teilweiser Deformation noch frei bewegen kann. In diesem Zustand ist eine einfache Ausrichtung des Kontaktglases gegenüber dem Auge möglich. Ist die gewünschte Justierlage erreicht, so wird der Abstand zwischen Kontaktglas und Patientenauge noch etwas verringert, wodurch der Ringspalt von der Hornhaut des Auges geschlossen ist und das Auge durch Unterdruck gegenüber dem Kontaktglas fixiert ist.

Die Krümmungsfläche, welche von der Linsenvorderfläche dem Auge als Soll-Form aufgeprägt ist, kann applikationsabhängig gewählt werden. Insbesondere sind auch asphärische Krümmungsflächen möglich, die es erlauben, optische Abbildungsfehler beim Einbringen von Behandlungs-Laserstrahlung zu minimieren. Auch kann die Linsenvorderfläche sphärisch mit einem Radius von 5-30 mm sein. Ein Radius der etwas geringer ist als der der menschlichen Hornhaut, also z. B. zwischen 15 und 25 mm ist besonders bevorzugt. Diese Sphärizität ist durch die erfindungsgemäße Ausgestaltung ohne Probleme möglich, da, anders als beim Stand der Technik in Form der US 2001/0021844 A1, keine Gefahr besteht, dass die Augenhornhaut den Unterdruck-Saugkanal so verschließt, dass nur eine unvollständige Befestigung des Kontaktglases am Auge erfolgt.

Vorzugsweise sind die die Linsenvorderfläche ringförmig umgebenden Ansaugöffnungen als Teil eines Saugkanals gebildet. Weiter ist günstigerweise die Ausbildung so, dass bei vollständig an die Linsenvorderfläche angelegtem Auge die Ansaugöffnungen noch nicht von der Augenhornhaut überdeckt sind, mithin keine Ansaugung erfolgt. In diesem Zustand kann dann eine genaue Ausrichtung des Kontaktglases gegenüber dem Auge erfolgen. Erst wenn das Kontaktglas weiter auf das Auge hingedrückt wird, überdeckt die Augenhornhaut auch die Ansaugöffnungen, wodurch die Unterdruckbefestigung wirkt.

Diese Ausgestaltung des Kontaktglases kann beispielsweise dadurch erreicht werden, dass in dem ringförmigen Bereich, in dem die Ansaugöffnungen liegen, eine Schrägstellung gegenüber der optischen Achse und/oder konkave Krümmung vorgesehen ist, z. B. eine, die etwas geringer ist, als die Krümmung der Augenhornhaut. Auch kann natürlich die Linsenvorderfläche konkav sein.

Ein zweckmäßiger und besonders einfach zu fertigender Aufbau des Kontaktglases besteht darin, dass die Linsenvorderfläche an einem Linsenkörper ausgebildet ist, der in einer Fassung gehalten ist, wobei die Ansaugöffnungen in der Fassung gebildet sind. Die eben genannte zweistufige Befestigung, bei der die Unterdruckbefestigung erst dann wirkt, wenn das Kontaktglas im justierten Zustand weiter auf das Auge hingedrückt wird, kann dann einfach dadurch erreicht werden, dass der axial vorderste Umriss der Fassung gegenüber einer Krümmung, die die Augenhornhaut hat, wenn das Auge vollständig an der Linsenvorderfläche anliegt, nicht vorsteht oder gegenüber dieser sogar zurückbleibt. Der Teil der Fassung, der die Ansaugöffnungen aufweist, wird zweckmäßigerweise möglichst nahtlos an die Linsenvorderfläche anschließen.

Eine besonders einfache Fertigung erreicht man, wenn der Saugkanal zwischen Fassung und Linsenkörper gebildet ist und Einzelkanäle vom Saugkanal durch eine Wandung der Fassung in die Ansaugöffnungen münden. Der Saugkanal kann dann beispielsweise ein ringförmiger Spalt zwischen Fassung und Linsenkörper sein, der patientenseitig von einer Wandung der Fassung überdeckt ist, die von einem außengelegen Rand der Fassung bis an den Rand der Linsenvorderfläche reicht. Durchbrüche in dieser Wandung bilden die Einzelkanäle und damit die Ansaugöffnungen.

Die Anzahl der Ansaugöffnungen ist zahlenmäßig nicht festgelegt, wird aber fertigungsbedingt entsprechend zu wählen sein. Die Form der Ansaugöffnungen kann auch nach Fertigungsgegebenheiten ausgewählt werden, insbesondere können sie rund, oval oder rechteckig sein. Um Verletzung der Augenhornhaut auszuschließen, sollte bei der Fertigung natürlich auf Gratfreiheit geachtet werden.

Die Krümmungsfläche, welche von der Linsenvorderfläche dem Auge als Soll-Form aufgeprägt ist, kann applikationsabhängig gewählt werden. Insbesondere sind auch asphärische Krümmungsflächen möglich, die es erlauben, optische Abbildungsfehler beim Einbringen von Behandlungs-Laserstrahlung zu minimieren.

Mit dem Kontaktglas kann die natürliche Form der Hornhaut bei angelegtem Kontaktglas gewahrt werden, was für Patienten besonders angenehm ist. Das Auge wird bei geeigneter Krümmung der Linsenvorderfläche mit minimalen Deformationen an das Kontaktglas gelegt. Die Linsenvorderfläche kann dabei sphärisch gekrümmt mit einem Krümmungsradius von 5-30 mm sein; bevorzugt ist ein Radius der etwas größer ist, als der des menschlichen Auges, und deshalb im Bereich von 15-25 mm liegt.

Die ringförmig liegenden Ansaugöffnungen vermeiden effektiv, dass Teile der Augenhornhaut bis in den oberen Bereich des Saugkanals gesaugt werden und diesen dadurch teilweise verstopfen. Die Einleitung des Unterdrucks in den Saugkanal ist folglich unkritisch, da immer der gesamte Saugkanal wirkt und insbesondere der Unterdruckanschluss nicht durch die Augenhornhaut verschlossen werden kann.

Das Kontaktglas ermöglicht, insbesondere wenn die Krümmungsfläche der Linsenvorderfläche etwas flacher gekrümmt ist, als die Augenhornhaut, ein besonders einfaches Aufsetzen des Kontaktglases auf die Augenhornhaut. Bei Annäherung an das Kontaktglas berührt zunächst nur der zentrale Bereich der Augenhornhaut, also der Augenhornhautscheitel, die Linsenvorderfläche. Bei zunehmendem Aufsetzen liegt nach und nach die Augenhornhaut an der vollständigen Linsenvorderfläche an, wobei auch in diesem Zustand die Ansaugöffnungen noch nicht abgedeckt sind und der Patient das Auge trotz teilweiser Deformation noch frei bewegen kann. In diesem Zustand ist eine einfache Ausrichtung des Kontaktglases gegenüber dem Auge möglich. Ist die gewünschte Justierlage erreicht, so wird der Abstand zwischen Kontaktglas und Patientenauge noch etwas verringert, wodurch der Ringspalt von der Hornhaut des Auges geschlossen ist und das Auge durch Unterdruck gegenüber dem Kontaktglas fixiert ist.

Es hat sich gezeigt, dass bei der Unterdruckbefestigung eines augenchirurgischen Kontaktglases das Ansaugen von Bindehaut (Sclera) an den Mitteln der Unterdruckbefestigung vermieden werden sollte, da ansonsten eine unzureichende Fixierung der Augenhornhaut, an der der chirurgische Eingriff erfolgen soll, auftreten kann. Die Unterdruckbefestigung wirkt also vorzugsweise ausschließlich an der Hornhaut und nicht der Sclera des Auges.

Die Erfindung sieht ganz unabhängig von der sonstigen Gestaltung des Kontaktglases und insbesondere unabhängig von der Gestaltung der Mittel zur Unterdruckbefestigung ein Kontaktglas für die Augenchirurgie vor, das eine zum Aufsetzen auf das Auge ausgebildete Linsenvorderfläche und eine Einrichtung zur Unterdruckbefestigung des Kontaktglases am Auge aufweist und weiter dadurch gekennzeichnet ist, dass am Kontaktglas ein einen geometrischen oder optischen Parameter des Kontaktglases kodierendes Kodierelement vorgesehen ist. Zweckmäßigerweise handelt es sich bei dem geometrischen oder optischen Parameter um den Durchmesser der Linsenvorderfläche. Das Kodierelement ist am Kontaktglas günstigerweise so angebracht, dass ein Anwender, d. h. ein Augenchirurg, von außen den gewünschten geometrischen oder optischen Parameter, beispielsweise den gewünschten Durchmesser der Linsenvorderfläche erkennen kann. Als Kodierelement kommt beispielsweise ein Strichcode, eine Ziffern- oder Buchstabenkennung oder auch ein geometrischer oder Farbcode in Frage. Besonders bevorzugt ist ein Kontaktglas, das eine Farbmarkierung trägt, wobei die Farbmarkierung dem Durchmesser der Linsenvorderfläche oder einem anderen geometrischen oder optischen Parameter des Kontaktglases zugeordnet ist. Fertigt man das Kontaktglas zweiteilig, d. h. aus einem die Linsenvorderfläche aufweisenden Linsenkörper sowie einer den Linsenkörper halternden Fassung, wird man das Kodierelement günstigerweise an der Fassung anordnen. Im Falle in der Farbcodierung kann beispielsweise die Fassung selbst eingefärbt werden.

Natürlich ist auch eine Variante möglich mit einem Informationsträger und mit einer Vorrichtung zum drahtlosen Auslesen und/oder Verändern der dort gespeicherten Information. Die Informationsübertragung erfolgt durch elektromagnetische Signale (z.B. im Radiofrequenzbereich zwischen 100kHz und 1GHz oder wie in WO 2005039462 A1 beschrieben).

Die in Verbindung mit dem Kontaktglas stehende Vorrichtung zum drahtlosen Auslesen und/oder Verändern der gespeicherten Information kann über einen Sender und Empfänger verfügen. Der Sender kann mit seinem Signal Energie an den Informationsspeicher übertragen, die dieser wiederum zur Aussendung von in ihm gespeicherter Information in Form eines Antwortsignals nutzt.

Es besteht die Möglichkeit mittels Signal vom Sender die im Informationsspeicher gespeicherte Information zu verändern. (Bei erneuter Informationsübertragung vom Zubehörteil zum Produkt wird dann die veränderte Information übertragen.) Der Informationsspeicher und die Fassung können eine mechanische Einheit bilden und es besteht die Möglichkeit, das Zubehörteil mit dem integrierten Informationsspeicher zu sterilisieren.

Damit ein Anwender ein Kontaktglas mit den gewünschten Parametern auswählen kann, ist die Kodierung vorteilhaft als optisch wahrnehmbare Kodierung ausgebildet, so dass ein Chirurg schnell und zielsicher das gewünschte Kontaktglas auswählen kann.

Die Erfindung wird nachfolgend unter Bezugnahme auf die Zeichnungen beispielhalber noch näher erläutert. In den Zeichnungen zeigt:
- Fig.1: eine schematische Darstellung einer Laserbearbeitungsvorrichtung für die Augenchirurgie,
- Fig. 2: eine schematische Darstellung der Augenhornhaut eines Patienten,
- Fig. 3 und 4: ein Kontaktglas für die Laserbearbeitungsvorrichtung der Fig. 1, wobei Fig. 3 eine Draufsicht und Fig. 4 eine Schnittdarstellung ist,
- Fig. 5 bis 8: Schema-Schnittdarstellungen von Kontaktgläsern ähnlich dem der Fig. 3/4,
- Fig.9: eine Schnittdarstellung einer weiteren Variante eines Kontaktglases für die Laserbearbeitungsvorrichtung der Fig. 1,
- Fig. 10: eine Draufsicht auf das Kontaktglas der Fig. 9 von unten (bezogen auf Fig. 3) und
- Fig. 11: eine Darstellung ähnlich der Fig. 10 für eine weitere Bauweise eines Kontaktglases.

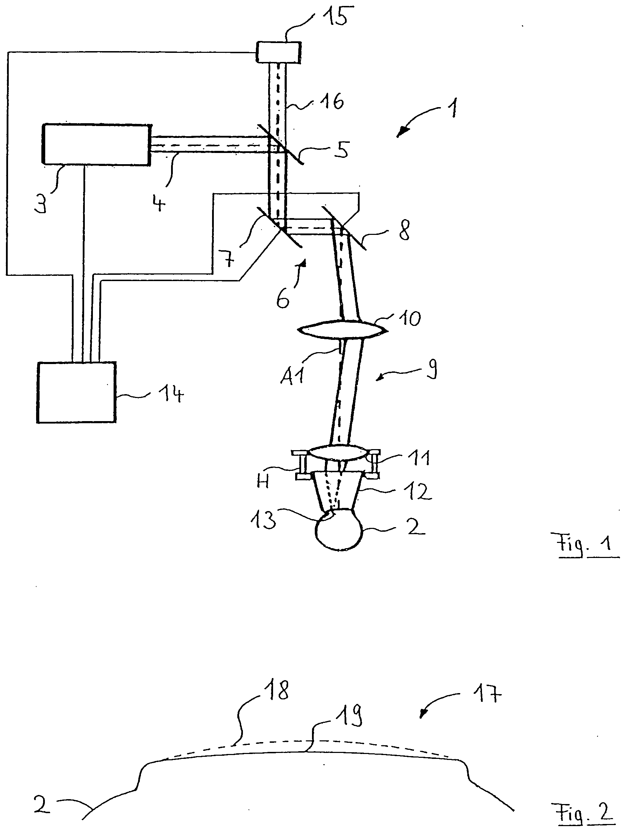

Fig. 1 zeigt ein Behandlungsgerät für ein augenchirurgisches Verfahren ähnlich dem in der EP 1159986 A1 bzw. der US 5549632 beschriebenen. Das Behandlungsgerät 1 der Figur 1 dient dazu, an einem Auge 2 eines Patienten eine Fehlsichtigkeitskorrektur gemäß dem bekannten Femtosekunden-LASIK-Verfahren auszuführen. Dazu weist das Behandlungsgerät 1 einen Laser 3 auf, der gepulste Laser-Strahlung abgibt. Die Pulsdauer liegt dabei z. B. im Femtosekundenbereich, und die Laserstrahlung wirkt mittels nichtlinearer optischer Effekte in der Hornhaut auf die eingangs beschriebene Art und Weise. Der vom Laser 3 entlang einer optischen Achse A1 abgegebene Behandlungsstrahl 4 fällt dabei auf einen Strahlteiler 5, der den Behandlungsstrahl 4 auf eine Scaneinrichtung 6 leitet. Die Scaneinrichtung 6 weist zwei Scanspiegel 7 und 8 auf, die um zueinander orthogonale Achsen drehbar sind, so dass die Scaneinrichtung 6 den Behandlungsstrahl 4 zweidimensional ablenkt. Eine verstellbare Projektionsoptik 9 fokussiert den Behandlungsstrahl 4 auf bzw. in das Auge 2. Die Projektionsoptik 9 weist dabei zwei Linsen 10 und 11 auf. Das Behandlungsgerät 1 stellt eine Laserbearbeitungsvorrichtung dar.

Der Linse 11 ist ein als Adapter wirkendes Kontaktglas 12 nachgeordnet, das über eine Halterung H fest mit der Linse 11 und damit dem Strahlengang des Behandlungsgerätes 1 verbunden ist. Das noch näher zu beschreibende Kontaktglas 12 liegt an der Hornhaut des Auges 2 an. Die optische Kombination aus Behandlungsgerät 1 mit daran befestigtem Kontaktglas 12 bewirkt, dass der Behandlungsstrahl 4 in einem in der Hornhaut des Auges 2 gelegenen Fokus 13 gebündelt wird.

Die Scaneinrichtung 6 wird ebenso wie der Laser 3 und die Projektionsoptik 9 über (nicht näher bezeichnete) Steuerleitungen von einem Steuergerät 14 angesteuert. Das Steuergerät 14 bestimmt dabei die Lage des Fokus 13 sowohl quer zur optischen Achse A1 (durch die Scanspiegel 7 und 8) sowie in Richtung der optischen Achse A1 (durch die Projektionsoptik 9).

Das Steuergerät 14 liest weiter einen Detektor 15 aus, der von der Hornhaut rückgestreute Strahlung, die den Strahlteiler 5 als Rückstrahlung 16 passiert, ausliest. Mittels des Detektors 15 kann der Betrieb des Lasers 3 sehr exakt gesteuert werden.

Das Kontaktglas 12 sorgt dafür, dass die Hornhaut des Auges 2 eine gewünschte Soll-Form erhält. Das Auge 2 befindet sich aufgrund der Anlage der Hornhaut 17 am Kontaktglas 12 in vorbestimmter Lage zum Kontaktglas 12 und somit zum damit verbundenen Behandlungsgerät 1.

Dies ist schematisch in Fig. 2 dargestellt, die einen Schnitt durch die Augenhornhaut 17 zeigt. Um eine exakte Positionierung des Fokus 13 in der Augenhornhaut 17 zu erreichen, muss die Krümmung der Augenhornhaut 17 berücksichtigt werden. Die Augenhornhaut 17 weist eine Ist-Form 18 auf, die von Patient zu Patient unterschiedlich ist. Der Adapter 12 liegt nun an der Augenhornhaut 17 derart an, dass er diese in eine gewünschte Soll-Form 19 verformt. Der genaue Verlauf der Soll-Form 19 hängt dabei von der Krümmung der dem Auge 2 zugewandten Linsenvorderfläche des Kontaktglases 12 ab. Durch den Adapter 12 sind bekannte geometrische und optische Verhältnisse für das Einbringen und Fokussieren des Behandlungsstrahls 4 in die Hornhaut 17 gegeben. Da die Hornhaut 17 am Kontaktglas 12 anliegt und dieses wiederum über die Halterung H gegenüber dem Strahlengang des Behandlungsgerätes 1 ortsfest ist, kann der Fokus 13 durch Ansteuerung der Scaneinrichtung 6 sowie der verstellbaren Projektionsoptik 9 dreidimensional exakt in der Hornhaut 17 positioniert werden.

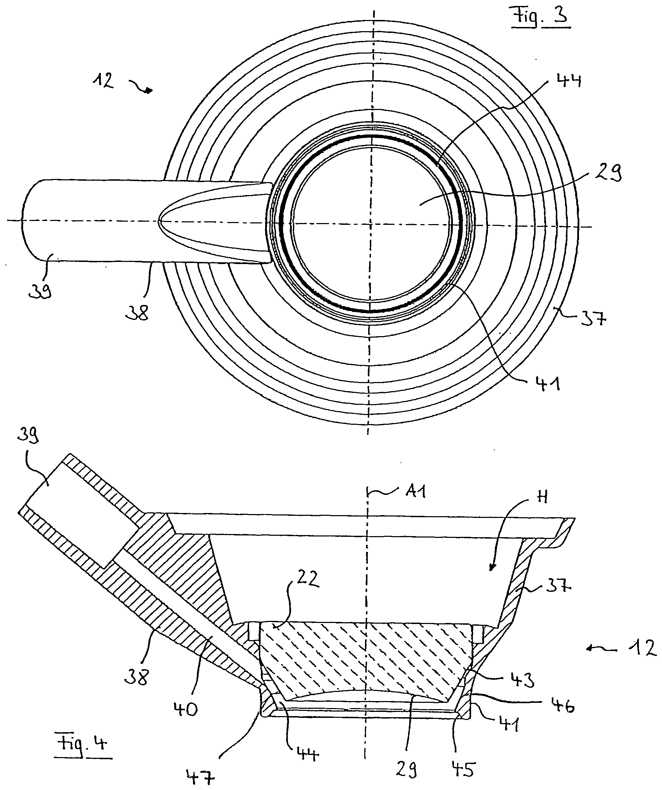

Die Fig. 3 und 4 zeigen eine Ausführungsform des Kontaktglases 12 im Detail; Fig. 4 ist eine Schnittdarstellung, Fig. 3 eine Ansicht des Kontaktglases 12 von vorne (in Fig. 4 von unten), d. h. in Sichtweite des Patienten. Das Kontaktglas 12 ist zweiteilig aufgebaut und besteht aus einem Linsenkörper 22, der in einer Fassung 37 befestigt ist, beispielsweise an einer Klebestelle 43 eingeklebt ist. Der Linsenkörper besteht aus Glas oder einem medizinisch zugelassenem Kunststoff, z.B. PMMA oder Polycarbonat. Diese Substanzen kommen auch für die Fassung in Frage, die zusätzlich noch aus Polyuretan oder Silikonkautschuk sein kann. Durch Andrücken des Kontaktglases 12 auf die Augenhornhaut 17 verleiht die Linsenvorderfläche 29 des in der Fassung 37 gehaltenen Linsenkörpers 22 der Augenhornhaut 17 die gewünschte Soll-Form 19.

Zum Bereitstellen des Unterdrucks weist die Fassung 37 einen Stutzen 38 auf, der über einen Luer-Lock-Anschluss 39 sowie eine im Stutzen verlaufende Unterdruck-Zuleitung 40 verfügt. Die Zuleitung 40 mündet seitlich des Linsenkörpers 22 oberhalb eines Ansatzringes 41 der Fassung 37. Zwischen einer dem Linsenkörper 22 zugewandten Ringinnenfläche 46 des Ansatzringes 41 und dem in dieser Ausführungsform kegelstumpfförmig ausgebildeten Linsenrand 47 ist ein Ringspalt 44 gebildet, der mit der Zuleitung 40 in Verbindung steht und als Saugkanal wirkt, durch den ringförmig Unterdruck auf die Augenhornhaut angebracht werden kann. Mittels Unterdruck am Luer-Lock-Anschluss 39 wird somit das Kontaktglas 12 so am Auge befestigt, dass die Hornhaut durch mechanischen Druck an die Linsenvorderfläche 29 angelegt wird, wodurch die Soll-Form 19 erreicht ist.

Der Ansatzring 41 steht bezogen auf die optische Achse A1 gegenüber der Linsenvorderfläche 29 vor.

Bei Aufsetzen des Kontaktglases auf die Augenhornhaut wird zuerst ein Kontakt zwischen der Linsenvorderfläche 29 und dem Hornhautscheitel hergestellt. Mit weiterem Anlegen der Augenhornhaut an die Linsenvorderfläche 29 wird in einem immer größerem Flächenbereich der Linsenvorderfläche ein Kontakt zur Augenhornhaut hergestellt. Liegt die Augenhornhaut vollständig an der Linsenvorderfläche 29 an, ist aufgrund der im Randbereich der Augenhornhaut stärker werdenden Krümmung der Augenhornhaut noch kein Kontakt zwischen der axial vordersten Umrisslinie des Ansatzringes 41 und der Augenhornhaut hergestellt, so dass der durch den Ringspalt 44 bereitgestellte Saugkanal noch nicht von der Augenhornhaut geschlossen ist. In diesem Zustand ist es also noch möglich, das Auge in Anlage an der Linsenvorderfläche 29 so zu justieren, dass die optische Achse A1 genau wie gewünscht liegt, beispielsweise mit der Sehachse übereinstimmt. Erst wenn Kontaktglas und Auge weiter zusammengedrückt werden, legt sich die Augenhornhaut auch an die axial vorderste Umrisslinie 45 des Ansatzringes 41 an, wodurch der Saugkanal am Ringspalt 44 geschlossen und das Kontaktglas 12 am Auge befestigt ist.

Die Fassung 37 ist also bezüglich des Ansatzringes 41 so gestaltet, dass die axial vorderste Umrisslinie 45 des Ansatzringes 41 nicht gegenüber der einer Krümmungsfläche, die durch die Krümmung des Auges bei vollständig an die Linsenvorderfläche 29 angelegter Augenhornhaut definiert ist, vorsteht. Der Ansatzring 41 liegt in einer Ausführungsform genau in der gedachten Verlängerung der Krümmungsfläche. In Vereinfachung kann auch Bezug auf die Krümmung der Linsenvorderfläche 29 genommen werden.

Durch die sich vom Auge spitzwinklig verjüngende Form des Ringspaltes 44 ist zuverlässig verhindert, dass Teile der Hornhaut bis in den Bereich des Zuleitung 40 gesaugt werden und diese dadurch zumindest teilweise verstopfen oder zulegen. Auch ist ein Verstopfen des Saugkanals 44 vermieden, so dass eine ringförmige Applikation des Unterdrucks sichergestellt ist.

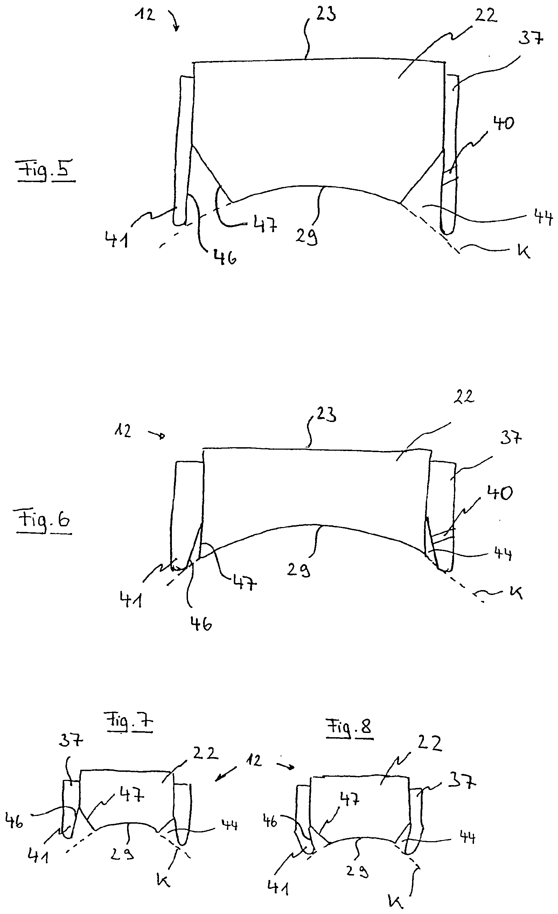

Die Fig. 5 bis 8 zeigen schematisch verschiedene Ausführungsformen der Geometrie von Linsenkörper 22 und Fassung 37. Elemente, die schon in der Bauweise der Fig. 3/4 vorhanden sind, sind mit denselben Bezugzeichen versehen, so dass diesbezüglich auch auf die Beschreibung zu den Figuren 3 und 4 verwiesen wird.

Wiederum weist das Kontaktglas 12 den Linsenkörper 22 auf, der in der Fassung 37 gehalten ist. Der Linsenkörper 22 wird begrenzt durch eine Eintrittsfläche 23 und die Linsenvorderfläche 29. Bevorzugt ist die Form einer plan-konkaven Linse, wobei die konkave Linsenvorderfläche 29 besonders bevorzugt hinsichtlich ihrer Krümmung der menschlichen Hornhaut entspricht, oder etwas flacher gekrümmt ist. Möglich sind jedoch auch andere Formen, insbesondere kann die Linsenvorderfläche 29 auch asphärisch sein, um optische Abbildungsfehler zu minimieren.

Die Fassung 37 umschließt den Linsenkörper 22 auf einem Teil ihres Umfanges, wodurch eine Verbindungsfläche zwischen Linsenkörper 22 und Fassungselement 37 im wesentlichen entlang einer zylindrischen Mantelfläche gebildet ist. Zwischen dem Rand des Linsenkörpers 22 und dem Ansatzring 41 der Fassung 37 und dem Rand 47 der Linsenvorderfläche 29 des Linsenkörpers 22 ist der als Saukanal dienende Ringspalt 44 gebildet.

Zur Ausführung dieses Ringspaltes kann nun, wie die Fig. 5 bis 8 zeigen, entweder die Ringinnenfläche 46 des Ansatzrings 41 und/oder der Linsenrand 47 konisch ausgebildet, d. h. schräg gegenüber der optischen Achse A1 verlaufend sein. In der Ausführungsform der Fig. 5 ist der Linsenrand 47 konisch, die Ringinnenfläche 46 zylindrisch. Weiter ist deutlich zu sehen, dass der Ansatzring 41 gegenüber der bereits genannten Krümmungsfläche K, die die Krümmung der angelegten Augenhornhaut vorgegeben ist, nicht vorsteht, sondern eher etwas zurückbleibt. Die Bauweise der Fig. 5 hat den Vorteil, dass eine einfache, im wesentlichen rohrförmige Fassung 37 verwendet werden kann. Weiter ist der Durchmesser der Linsenvorderfläche 29 kleiner, als der der Eintrittsfläche 23.

Den umgekehrten Fall zeigt die Fig. 6, hier ist der Linsenrand 47 zylindrisch, die Ringinnenfläche 46 hingegen konisch ausgebildet, wobei der Konus sich nun zur Krümmungsfläche K hin öffnet. Auch reicht hier der Ansatzring 41 exakt bis an die Krümmungsfläche K.

In der Bauweise gemäß Fig. 7 sind sowohl der Linsenkörper 22 am Linsenrand 47 konisch (zum Auge verjüngend) als auch die Ringinnenfläche 46 (zum Auge hin aufweitend). Weiter bleibt die axial vorderste Umrisslinie des Ansatzringes 41 gegenüber der Krümmungsfläche K zurück. Dies hat den Vorteil, dass ein vollständiges Justieren des Auges gegenüber dem Kontaktglas 12 möglich ist und erst bei zusätzlich aufgebrachten mechanischem Druck der Saugkanal durch Anlage der Augenhornhaut geschlossen wird.

Fig. 8 schließlich zeigt eine Bauweise ähnlich der Fig. 7, jedoch sind sowohl Ringinnenfläche 46 als auch Linsenrand 47 konisch zum Auge verjüngt ausgebildet. Vorteilhaft ist in diesem Fall der kleinere Durchmesser der Auflagefläche des Kontaktglases 12 auf dem Auge bei gleichzeitig großem Durchmesser der Eintrittsfläche 23.

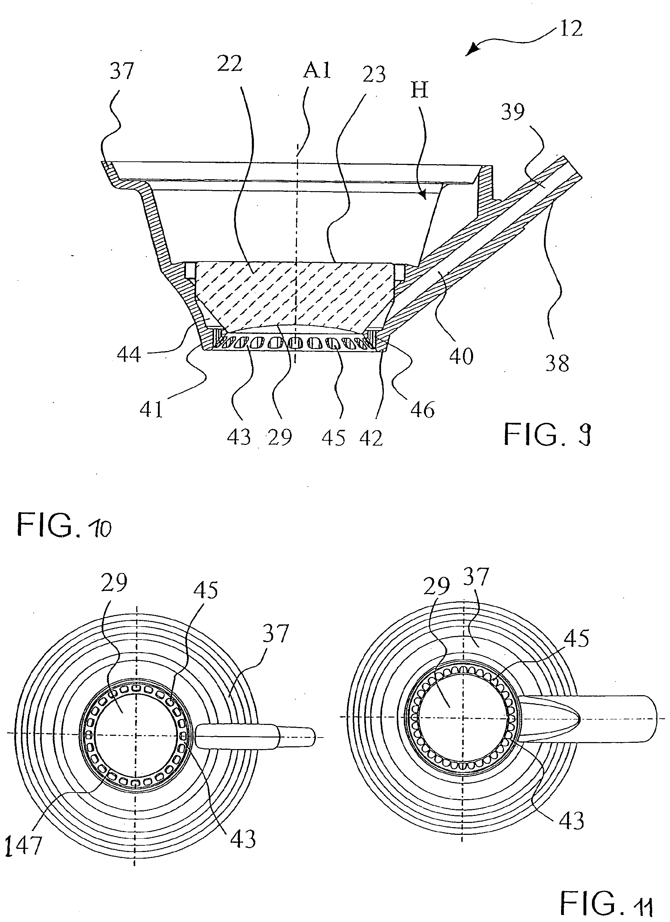

Fig. 9 zeigt eine Schnittdarstellung einer weiteren Variante des Kontaktglases 12 im Detail. Es handelt sich dabei um eine Abwandlung des Kontaktglases der Fig. 3 und 4, weshalb gleiche Elemente mit den gleichen Bezugzeichen versehen wurden. Das Kontaktglas 12 ist zweiteilig aufgebaut und besteht aus dem Linsenkörper 22, der in einer Fassung 37 befestigt, beispielsweise eingeklebt ist. Der Linsenkörper 22, der beispielsweise aus Glas ausgebildet sein kann, hat die planare Eintrittsfläche 23, an der die Behandlungsstrahlung vom Laserbehandlungsgerät 1 zugeführt wird, und die gegenüberliegende, patientenseitig gelegenen Linsenvorderfläche 29, die gekrümmt und der Krümmung der menschlichen Hornhaut angepasst ist. Durch Andrücken des Kontaktglases 12 auf die Augenhornhaut 17 verleiht die Linsenvorderfläche 29 des in der Fassung 37 gehaltenen Linsenkörpers 22 der Augenhornhaut 17 die gewünschte Sollform 19. Der Linsenkörper 22 besteht aus Glas oder medizinisch zugelassenem Kunststoff, z. B. PMMA oder Polycarbonat. Diese Materialien kommen auch für die Fassung in Frage, die zusätzlich noch aus Polyuretan oder Silikonkautschuk sein kann.

Zum Bereitstellen des Unterdrucks weist die Fassung 37 den Stutzen 38 auf, der über den Anschlussstutzen 39, auf den ein Unterdruckschlauch aufgeschoben wird, sowie die im Stutzen 38 verlaufende Unterdruckzuleitung 40 verfügt. Die Zuleitung 40 mündet seitlich des Linsenkörpers 22 oberhalb des Ansatzringes 41 der Fassung 37.

Der Ansatzring 41 setzt mit seiner patientenseitig gelegenen Fläche (in der Darstellung der Fig. 3 von unten gesehen) die Krümmung der Linsenvorderfläche 29 fort, so dass sein axial äußerster Umriss in Form des unteren Randes 42 in einer Verlängerung der Krümmung der Linsenvorderfläche 29 liegt. Zwischen diesem Rand 42 und dem Außenrand der Linsenvorderfläche 29 ist die Ansaugfläche 43 gebildet.

Die Ansaugfläche 43 überdeckt den ringförmigen Saugkanal 44, der in dieser Ausführungsform durch einen Spalt zwischen Fassung 37 und Linsenköper 22 gebildet ist. Prinzipiell wäre aber auch ein Saugkanal 44, der vollständig im Material der Fassung 37 liegt, möglich. In der in Fig. 9 gezeigten Ausführungsform ist die Ansaugfläche 37 also durch eine Wandung gebildet, die vom äußersten Rand 42 des Ansatzringes 41 zum Rand der Linsenvorderfläche 29 hin führt. In der Ansaugfläche 43, d. h. in dieser Wandung, sind Ansaugöffnungen 45 gebildet, die das patientenseitig gelegene Ende von Einzelkanälen 46 sind, welche an ihrem anderen Ende in den Saugkanal 44 münden.

Am Unterdruck-Anschluss 39 angelegter Unterdruck gelangt also durch die Unterdruckzuleitung 40 in den Saugkanal 44 und wirkt dort durch die ringförmig die Linsenvorderfläche 29 umgebenden Ansaugöffnungen 45 auf die Augenhornhaut.

Da die Ansaugfläche 43 die Krümmung der Linsenvorderfläche 29 bevorzugt glatt fortsetzt (wobei eine Fortsetzung einer sphärischen Krümmungsfläche möglich ist, jedoch auch eine asphärische Krümmung oder eine Krümmung mit einem gegenüber der Linsenvorderfläche 29 abweichenden, größeren oder kleineren Krümmungsradius), ist insgesamt ein weitgehend fließender Übergang von der Linsenvorderfläche 29 auf die Ansaugfläche 43 gegeben. Auf jeden Fall hat der Übergang keine scharfen Kanten sondern besteht höchstens in einer ringförmigen Nahtstelle, an der die Krümmung oder Neigung wechselt.

Fig. 10 zeigt das Kontaktglas 12 der Fig. 9 in einer Ansicht von der Patientenseite her, also in Fig. 9 von unten. Wie gut zu sehen ist, sind die Ansaugöffnungen 45 hier oval ausgestaltet und umgeben die Linsenvorderfläche 29 außerhalb einer Nahtstelle 147 zwischen der Fassung 37 und dem Linsenkörper 22 im Bereich der ringförmigen Ansaugfläche 43.

Fig. 11 zeigt eine abgewandelte Bauweise, wobei hier die Ansaugöffnungen 45 geometrisch anders gestaltet sind, nämlich in Form von halben Ovalen, die eine arkadenförmige Struktur der Ansaugöffnungen 45 ergeben. Auch ist hier der UnterdruckAnschluss anders gestaltet, nämlich als Luer-Lock-Anschluss.

Der Durchmesser der ringförmigen Ansaugfläche 43 und damit auch der Linsenvorderfläche 29 ist vorzugsweise patientenabhängig gewählt. Es sind also für ein Behandlungsgerät verschiedene Kontaktgläser 12 mit unterschiedlichen Durchmessern der Linsenvorderfläche 29 und damit Durchmesser der ringförmigen Ansaugfläche 43 vorgehalten, so dass die Ansaugöffnungen 45 in jedem Fall auf der Cornea des zu behandelnden Patienten aufsitzen und somit eine optimale Ansaugung gewährleistet ist. Eine Ansaugung von Bindehaut im Bereich der Ansaugfläche 43 ist somit ausgeschlossen. Um dem Anwender die Unterscheidung zwischen verschiedenen Kontaktgläsern 12, beispielsweise von Kontaktgläsern mit verschiedenen Durchmessern der Linsenvorderfläche 29 (und damit Ansaugfläche 43) oder mit verschiedenen Krümmungsradien zu erleichtern, ist eine Kodierung im Bereich der Fassung 37 vorgesehen.

Dazu ist die Fassung mit einem RFID-Chip ausgestattet, das Gerät 1 mit einer entsprechenden Sender/Empfänger-Einheit. Die Reichweite der Sender/Empfänger-Einheit ist räumlich eng begrenzt (z.B. 10 cm). Wird das Kontaktglas in die Halterung gesetzt, prüft das Gerät 1 die gespeicherte Information, wertet sie aus und verändert sie gegebenenfalls. Diese Veränderung ist bei einmalig zu verwendenden Kontaktgläsern vorteilhaft, weil dadurch verhindert werden kann, dass eine wiederholte Verwendung erfolgt.

Konkret kann der RFID-Chip eingegossen werden. Der RFID-Chip kann einzeln oder auch im Zusammenhang mit dem ganzen Kontaktglas sterilisiert werden. Zu bevorzugen ist eine Gassterilisation mit Ethylen-Dioxid (ETO). Das Behandlungsgerät 1 ist mit einem RFID-Sender-Empfänger ausgerüstet und liest die gespeicherte Information (z.B. eines ID- oder Benutzungscodes). Eine mehrmalige Verwendung ist entweder dadurch verhindert, dass der gleiche Benutzungscode nur einmal vom jeweiligen Gerät 1 akzeptiert wird, d.h. das Gerät 1 verändert die Information im Chip so, dass eine zweite Verwendung auch an anderen Geräten ausgeschlossen ist.

Dieses Prinzip kann natürlich für beliebige Kombinationen aus einem Zubehörteil und einem medizintechnischen Gerät eingesetzt werden.

Um beim Einsatz des Kontaktglases 12 die Beobachtung des Patientenauges zu erleichtern, ist es möglich, durch die Fassung 37 hindurch Licht von einer Lichtquelle zum Behandlungsort einzustrahlen. Dies ist beispielsweise in der DE 10353264 A1 beschrieben. Die nun lediglich im Bereich der Ansaugöffnungen 45 durchbrochene Ansaugfläche 43 erleichtert dabei die Einkopplung der Strahlung erheblich und die in der DE 10353264 A1 beschriebenen optischen Mittel, die zum Ausgleichen der optischen Wirkung eines ringförmig offenen Saugkanals vorgesehen sind, müssen nicht mehr angewandt werden.

Bei Aufsetzen des Kontaktglases auf die Augenhornhaut wird zuerst ein Kontakt zwischen der Linsenvorderfläche 29 und dem Hornhautscheitel hergestellt. Mit weiterem Anlegen der Augenhornhaut an die Linsenvorderfläche 29 wird in einem immer größerem Flächenbereich der Linsenvorderfläche ein Kontakt zur Augenhornhaut hergestellt. Liegt die Augenhornhaut vollständig an der Linsenvorderfläche 29 an, ist aufgrund der im Randbereich der Augenhornhaut stärker werdenden Krümmung der Augenhornhaut noch kein Kontakt zwischen der axial vordersten Umrisslinie des Ansatzringes 41 und der Augenhornhaut hergestellt, so dass die Ansaugöffnung 45 noch nicht von der Augenhornhaut überdeckt sind. In diesem Zustand ist es also noch möglich, das Auge in Anlage an der Linsenvorderfläche 29 so zu justieren, dass die optische Achse A1 genau wie gewünscht liegt, beispielsweise mit der Sehachse übereinstimmt. Erst wenn Kontaktglas und Auge weiter zusammengedrückt werden, legt sich die Augenhornhaut auch an die axial vorderste Umrisslinie 42 des Ansatzringes 41 an, wodurch der Saugkanal 44 an den Ansaugöffnungen 45 geschlossen und das Kontaktglas 12 am Auge befestigt ist.

Die Fassung 37 ist z. B. bezüglich des Ansatzringes 41 so gestaltet, dass die axial vorderste Umrisslinie des Randes 42 des Ansatzringes 41 nicht gegenüber der einer Krümmungsfläche, die durch die Krümmung des Auges bei vollständig an die Linsenvorderfläche 29 angelegter Augenhornhaut definiert ist, vorsteht. Der Rand 42 liegt in einer Ausführungsform genau in der gedachten Verlängerung der Krümmungsfläche. In Vereinfachung kann auch Bezug auf die Krümmung der Linsenvorderfläche 29 genommen werden.

Natürlich können die hier geschilderten geometrischen Ausgestaltungen auch einzeln oder in anderen, nicht ausdrücklich gezeigten oder beschriebenen Kombinationen vorteilhaft eingesetzt werden.

Natürlich können die hier geschilderten geometrischen Ausgestaltungen auch einzeln oder in anderen, nicht ausdrücklich gezeigten oder beschriebenen Kombinationen vorteilhaft eingesetzt werden.

## Patentansprüche

1. System aus einem eine Sende/Lese-Einheit aufweisenden Behandlungsgerät (1) und einem Kontaktglas für die Augenchirurgie, das eine zum Aufsetzen auf das Auge (2) ausgebildete Linsenvorderfläche (29) und eine Einrichtung zur Unterdruckbefestigung des Kontaktglases (12) am Auge (2) aufweist, wobei am Kontaktglas (12) ein einen geometrischen oder optischen Parameter kodierendes Kodierelement vorgesehen ist, das ein einen ID-Code speichernder RFID-Chip ist, der zur Zusammenwirkung mit der Sende/Lese-Einheit ausgebildet ist, und wobei die Sende/Lese-Einheit zur Auswertung des ID-Codes, welchen der RFID-Chip sendet, eingerichtet ist und der Betrieb des Behandlungsgerätes (1) abhängig von diesem ID-Code erfolgt, wobei der Betrieb nur bei bestimmtem ID-Code möglich ist, wobei die Sende/Lese-Einheit zur Veränderung oder Löschung des ID-Codes im RFID-Chip nach einer Verwendung des Kontaktglases eingerichtet ist.

2. System nach Anspruch 1, **dadurch gekennzeichnet, dass** die Linsenvorderfläche (29) des Kontaktglases (12) ringförmig von einem gegenüber einer gedachten Verlängerung der Linsenvorderfläche nicht vorstehenden Ringspalt (44) oder von einer Vielzahl von Ansaugöffnungen (45) umgeben ist, durch den/die hindurch Unterdruck auf das Auge (2) bewirkbar ist.

3. System nach Anspruch 2, **dadurch gekennzeichnet, dass** das Kontaktglas (12) einen den Rand der Linsenvorderfläche (29) ringförmig umgebenden Saugkanal (44) aufweist, in den die Ansaugöffnungen (45) münden.

4. System nach Anspruch 2 oder 3, **dadurch gekennzeichnet, dass** die Linsenvorderfläche (29) an einem Linsenkörper (22) ausgebildet ist, der in einer Fassung (37) gehalten ist, wobei die Ansaugöffnungen (45) in der Fassung (37) gebildet sind.

5. System nach Anspruch 3, **dadurch gekennzeichnet, dass** der Saugkanal (44) zwischen Fassung (37) und Linsenkörper (22) gebildet ist und dass vom Saugkanal (44) durch eine Wandung der Fassung (37) hindurch in die Ansaugöffnungen (45) mündende Einzelkanäle (46) gebildet sind.

6. System nach Anspruch 4 oder 5, **dadurch gekennzeichnet, dass** die Linsenvorderfläche (29) konkav gekrümmt ausgebildet ist und die Ansaugöffnungen (45) ebenfalls auf einer gekrümmten Ringfläche liegen.

7. System nach einem der Ansprüche 2 bis 6, **dadurch gekennzeichnet, dass** die Ansaugöffnungen (45) rund, oval oder rechteckig sind.

8. System nach Anspruch 2, **dadurch gekennzeichnet, dass** das Kontaktglas (12) einen in einer Fassung (37) aufgenommenen Linsenkörper (22) aufweist, welcher eine zum Aufsetzen auf das Auge (2) ausgebildete, konkave Linsenvorderfläche (29) aufweist, wobei am Rand der Linsenvorderfläche (29) zwischen dem Linsenkörper (22) und der Fassung (37) der Ring-Spalt (44) gebildet ist, der zusammen mit dem angelegten Auge (2) als Saugkanal wirkt, über den zum Befestigen des Kontaktglases (12) am Auge (2) Unterdruck applizierbar ist, wobei die Fassung (37) so ausgebildet ist, dass bei vollflächig an die Linsenvorderfläche (29) angelegtem Auge (2) der Saugkanal zwischen Ringspalt (44) und Auge (2) noch nicht geschlossen ist.

9. System nach Anspruch 8, **dadurch gekennzeichnet, dass** die Linsenvorderfläche (29) gemäß einer Krümmungsfläche (K) konkav ausgebildet ist und der axial zum Auge (2) hin vorderste Umriß der Fassung (37) in gedachter Verlängerung der Krümmungsfläche (K) liegt oder zumindest gegenüber dieser nicht vorsteht.

10. System nach Anspruch 9, **dadurch gekennzeichnet, dass** eine den Ring-Spalt (44) begrenzende Innenfläche der Fassung (37) und/oder eine den Ring-Spalt (44) begrenzende Randfläche (47) des Linsenkörpers (22) als schräg zur optischen Achse (A1) verlaufende Kegelstumpffläche ausgebildet ist.

11. System nach einem der Ansprüche 8 bis 10, **dadurch gekennzeichnet, dass** der Ring-Spalt (44) sich vom Auge (2) weg verjüngt.

12. System nach Anspruch 11, **dadurch gekennzeichnet, dass** das vom Auge (2) abgewandte Ende des Ring-Spaltes (44) unter einem Winkel ≤ 90° zusammenläuft.

13. System nach einem der obigen Ansprüche 2 bis 12, **dadurch gekennzeichnet, dass** das Kontaktglas (12) Mittel zum Einkoppeln von Beleuchtungsstrahlung im Bereich der ringförmig angeordneten Ansaugöffnungen (45) aufweist.

14. System nach einem der obigen Ansprüche, **dadurch gekennzeichnet, dass** die Linsenvorderfläche (29) asphärisch ist.

15. System nach einem der obigen Ansprüche, **dadurch gekennzeichnet, dass** der Parameter ein Durchmesser der Linsenvorderfläche (29) des Kontaktglases (12) ist.

16. System nach Anspruch 2, **dadurch gekennzeichnet, dass** der Ringspalt (44) oder die Ansaugöffnungen (45) so ausgebildet sind, dass der Unterdruck auf die Hornhaut (17) des Auges (2) wirkt.

17. System nach Anspruch 1, **dadurch gekennzeichnet, dass** die Sende/Lese-Einheit den ID-Code im RFID-Chip nach einer Verwendung des Kontaktglases ungültig macht.

## Claims

1. A system comprising a treatment apparatus (1) including a transmitting/reading unit and a contact glass for ophthalmic surgery including an anterior lens surface (29) for placement onto the eye (2) and means for vacuum fixation of the contact glass (12) to the eye (2),
wherein the contact glass (12) comprises a coding element which codes a geometric or optical parameter, said coding element being an RFID chip storing an ID code, said RFID chip being adapted to cooperate with the transmitting/reading unit to evaluate the ID code sent from the RFID chip, wherein operation of the treatment apparatus (1) depends on this ID code, wherein operation is only possible at certain ID codes, wherein the transmitting/reading unit is adapted to change or delete the ID code in the RFID chip after the contact glass was used.

2. The system according to claim 1, **characterized in that** the anterior lens surface (29) of the contact glass (12) is annularly surrounded by an annular gap (44) which does not protrude with respect to an imaginary extension of the anterior lens surface or by a plurality of suction orifices (45) through which the vacuum is applicable to the eye (2).

3. The system according to claim 2, **characterized in that** the contact glass (12) comprises a suction channel (44), which annularly surrounds the periphery of the anterior lens surface (29) and to which the suction orifices (45) open.

4. The system according to claim 2 or 3, **characterized in that** the anterior lens surface (29) is formed on a lens body (22) held in a mount (37), the suction orifices (45) being formed in the mount (37).

5. The system according to claim 3, **characterized in that** the suction channel (44) is formed between the mount (37) and the lens body (22) and that individual channels (46) are formed, extending from the suction channel (44) through a wall of the mount (37) into the suction orifices (45).

6. The system according to claim 4 or 5, **characterized in that** the anterior lens surface (29) is provided with a concave curvature and the suction orifices (45) are also located on a curved annular surface.

7. The system according to any of claims 2 to 6, **characterized in that** the suction orifices (45) are round, oval or rectangular.

8. The system according to claim 2, **characterized in that** the contact glass (12) comprises a lens body (22), which is held in a mount (37) and comprises a concave anterior lens surface (29) adapted to be placed on the eye (2), with the annular gap (44) being formed at the edge of the anterior lens surface (29) between the lens body (22) and the mount (37), which gap, together with the contacted eye (2), acts as a suction channel via which a vacuum is applicable for fixing the contact glass (12) to the eye (2), with the mount (37) being provided such that the suction channel between the annular gap (44) and the eye (2) is not yet closed when the eye (2) is placed in full-surface contact with the anterior lens surface (29).

9. The system according to claim 8, **characterized in that** the anterior lens surface (29) is concave according to a curved surface (K) and the contour of the mount (37), being axially foremost towards the eye (2), is located in an imaginary extension of the curved surface (K) or at least does not protrude with respect to the latter.

10. The system according to claim 9, **characterized in that** an internal surface of the mount (37), which limits the annular gap (44), and/or a rim surface (47) of the lens body (22), which limitis the annular gap (44), is formed as a frusto-conical surface extending obliquely with respect to the optical axis (A1).

11. The system according to any of claims 8 to 10, **characterized in that** the annular gap (44) tapers away from the eye (2).

12. The system according to claim 11, **characterized in that** the bottom of the annular gap (44) facing away from the eye (2) tapers at an angle of ≤ 90°.

13. The system according to any of claims 2 to 12, **characterized in that** the contact glass (12) comprises means for coupling illumination radiation into the region of the annularly arranged suction orifices (45).

14. The system according to any of the above claims, **characterized in that** the anterior lens surface (29) is aspherical.

15. The system according to any of the above claims, **characterized in that** the parameter of the contact glass (12) is a diameter of the anterior lens surface (29) of the contact glass (12).

16. The system according to claim 2, **characterized in that** the annular gap (44) or the suction orifices (45) are provided such that the vacuum acts on the cornea (17) of the eye (2).

17. The system according to claim 1, **characterized in that** the transmitting/reading unit invalidates the ID code in the RFID chip after the contact glass was used.

## Revendications

1. Système constitué d'un appareil de traitement (1) comportant une unité d'émission/lecture et d'un verre de contact destiné à la chirurgie oculaire, comportant une face avant de lentille (29) devant être posée sur l'oeil (2) et un dispositif destiné à fixer par dépression le verre de contact (12) sur l'oeil (2),
dans lequel il est prévu sur le verre de contact (12) un élément de codage codant un paramètre géométrique ou optique, qui est une puce RFID stockant un code ID réalisée de manière à coopérer avec l'unité d'émission/lecture et dans lequel l'unité d'émission/lecture est conçue pour évaluer le code ID qui est émis par la puce RFID, le fonctionnement de l'appareil de traitement (1) s'effectuant en fonction dudit code ID, dans lequel le fonctionnement n'est possible que pour des codes ID déterminés,
dans lequel l'unité d'émission/lecture est conçue pour modifier ou effacer le code ID contenu dans la puce RFID après une utilisation du verre de contact.

2. Système selon la revendication 1, **caractérisé en ce que** la face avant de lentille (29) du verre de contact (12) est entourée de manière annulaire par une fente annulaire (44) qui n'est pas présente par rapport à un prolongement imaginaire de la face avant de lentille ou par une pluralité d'ouvertures d'aspiration (45) au moyen desquelles une dépression peut être exercée sur l'oeil (2).

3. Système selon la revendication 2, **caractérisé en ce que** le verre de contact (12) comporte un canal d'aspiration (44) entourant de manière annulaire le bord de la face avant de lentille (29), canal d'aspiration dans lequel débouchent les ouvertures d'aspiration (45).

4. Système selon la revendication 2 ou 3, **caractérisé en ce que** la face avant de lentille (29) est formée sur un corps de lentille (22) qui est maintenu dans une monture (37), dans lequel les ouvertures d'aspiration (45) sont formées dans la monture (37).

5. Système selon la revendication 3, **caractérisé en ce que** le canal d'aspiration (44) est formé entre la monture (37) et le corps de lentille (22) et **en ce que** des canaux individuels (46) partant du canal d'aspiration (44) et débouchant dans les orifices d'aspiration (45) sont formés à travers une paroi de la monture (37).

6. Système selon la revendication 4 ou 5, **caractérisé en ce que** la face avant de lentille (29) est réalisée de manière à présenter une courbure concave et **en ce que** les ouvertures d'aspiration (45) se situent également sur une surface annulaire incurvée.

7. Système selon l'une quelconque des revendications 2 à 6, **caractérisé en ce que** les ouvertures d'aspiration (45) sont de forme arrondie, ovale ou rectangulaire.

8. Système selon la revendication 2, **caractérisé en ce que** le verre de contact (12) comporte un corps de lentille (22) reçu dans une monture (37) qui présente une face avant de lentille (29) concave formée de manière à être placée sur l'oeil (2), dans lequel la fente annulaire (44) est formée sur le bord de la face avant de lentille (29) entre le corps de lentille (22) et la monture (37), laquelle fente agit en association avec l'oeil (2) soumis à l'application en tant que canal d'aspiration par l'intermédiaire duquel une dépression peut être appliquée pour fixer le verre de contact (12) à l'oeil (2), dans lequel la monture (37) est réalisée de manière à ce que le canal d'aspiration formé entre la fente annulaire (44) et l'oeil (2) ne soit pas encore fermé lors d'une application à l'oeil (2) sur la totalité de la surface de la face avant de lentille (29).

9. Système selon la revendication 8, **caractérisé en ce que** la face avant de lentille (29) est réalisée de manière à être concave conformément à une surface de courbure (K) et **en ce que** le profil le plus en avant de la monture (37) axialement par rapport à l'oeil (2) se situe dans le prolongement imaginaire de la surface de courbure (K) ou ne fait au moins pas saille par rapport audit prolongement.

10. Système selon la revendication 9, **caractérisé en ce qu'**une surface interne délimitant la fente annulaire (44) de la monture (37) et/ou une surface de bord (47) délimitant la fente annulaire (44) du corps de lentille (22) est/sont réalisé(s) sous la forme de surfaces tronconiques s'étendant en biais par rapport à l'axe optique (A1).

11. Système selon l'une quelconque des revendications 8 à 10, **caractérisé en ce que** la fente annulaire (44) se rétrécit en s'éloignant de l'oeil (2).

12. Système selon la revendication 11, **caractérisé en ce que** l'extrémité de la fente annulaire (44) tournée en sens opposé à l'oeil (2) converge sous un angle ≤ 90°.

13. Système selon l'une quelconque des revendications 2 à 12 précédentes, **caractérisé en ce que** le verre de contact (12) comporte des moyens destinés à injecter un rayonnement d'éclairage dans la zone des ouvertures d'aspiration (45) disposées de manière annulaire.

14. Système selon l'une quelconque des revendications précédentes, **caractérisé en ce que** la face avant de lentille (29) est asphérique.

15. Système selon l'une quelconque des revendications précédentes, **caractérisé en ce que** le paramètre est un diamètre de la face avant de lentille (29) du verre de contact (12).

16. Système selon la revendication 2, **caractérisé en ce que** la fente annulaire (44) ou les ouvertures d'aspiration (45) sont réalisées de manière à ce que la dépression agisse sur la cornée (17) de l'oeil (2).

17. Système selon la revendication 1, **caractérisé en ce que** l'unité d'émission/lecture rendent inutilisable le code ID contenu dans la puce RFID après une utilisation du verre de contact.
